(19) Europäisches Patentamt
European Patent Office
Office européen des brevets

(11) Publication number: 0 194 688 B1

(12) EUROPEAN PATENT SPECIFICATION

(45) Date of publication of patent specification: 18.07.90

(51) Int. Cl.$^5$: C 07 C 275/46, A 01 N 47/34

(21) Application number: 86103412.2

(22) Date of filing: 13.03.86

# (54) Benzoyl-ureas having insecticide activity.

(30) Priority: 14.03.85 IT 1991585

(43) Date of publication of application:
17.09.86 Bulletin 86/38

(45) Publication of the grant of the patent:
18.07.90 Bulletin 90/29

(84) Designated Contracting States:
AT BE CH DE FR GB LI NL SE

(56) References cited:
US-A-4 350 706

(73) Proprietor: ISTITUTO GUIDO DONEGANI S.p.A.
Via Caduti del Lavoro
I-28100 Novara (IT)

(72) Inventor: Massardo, Pietro
5, via Fra' Bartolomeo
I-20123 Milan (IT)
Inventor: Rama, Franco
1, via Rodi
I-21052 Busto Arsizio Varese (IT)
Inventor: Piccardi, Paolo
8, via E. De Marchi
I-20125 Milan (IT)
Inventor: Caprioli, Vincenzo
8, via Loriga
I-27028 S. Martino Siccomario Pavia (IT)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 194 688 B1

Courier Press, Leamington Spa, England.

## Description

The present invention relates to benzoylurea derivatives having insecticidal activity and, in particular, it relates to 1-benzoyl-3-aryl-urea derivatives which are particularly active against eggs and larvae of insects noxious in the agrarian and civil field, as well as to the use of these derivatives and a process for their preparation.

Several 1-benzoyl-3-aryl-urea derivatives having insecticide activity are known.

Among them, Diflubenzuron (generic name for 1-(2,6-difluorobenzoyl)-3-(4-chlorophenyl)-urea) which is described in US—A—3 993 908 has been the first product to be marketed. However, Diflubenzuron is suspected of being a carcinogen [European Chem. News *6* (16); 29 (1978)], as it contains a 4-chloroaniline unit in its molecule.

An object of the present invention are 1-benzoyl-3-aryl-urea derivatives having the general formula:

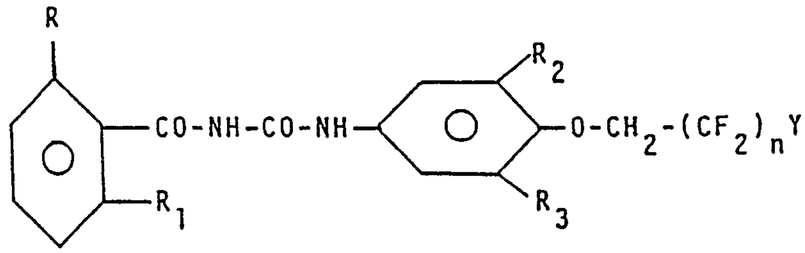

(I)

wherein:

R represents chlorine or fluorine;

$R_1$ represents hydrogen, chlorine or fluorine;

$R_2$ and $R_3$, which are the same or different from each other, represent hydrogen or halogen;

Y is hydrogen or fluorine; and

n is an integer of from 1 to 4.

Among the aforesaid definitions the term halogen preferably refers to a fluorine, chlorine, or bromine atom. The compounds of formula (I) exhibit insecticide activity and may be used in the agrarian, forestal, civil and veterinary field for combatting insect infestations.

In the following description of the preparation of the compounds of formula (I) the symbols R, $R_1$, $R_2$, Y and n have the meanings indicated for formula (I), if not specified otherwise.

The compounds of formula (I) are obtained by reacting a benzoyl-isocyanate having the formula:

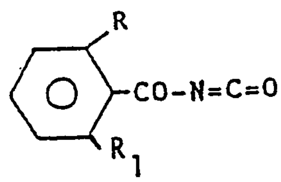

(II)

with an aromatic amine having the formula:

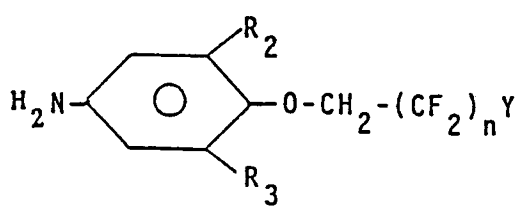

(III)

The reaction does not require the presence of catalysts and is carried out in an inert solvent at a temperature ranging between 0°C and the boiling temperature of the mixture.

Suitable solvents are e.g. aromatic hydrocarbons, chlorinated hydrocarbons, ethers, ketones and acetonitrile.

The benzoyl-isocyanates of formula (II) are known compounds, some of them being commercially available.

The amines of formula (III) may be prepared in a manner known per se, for instance by:

a) reaction of a sodium or potassium salt of a suitable amino-phenol (IV) in a dipolar aprotic solvent with a compound of formula:

$$X—CH_2—(CF_2)_nY \qquad (V)$$

wherein X is halogen such as Cl, Br, J or a tosyl or mesyl group, at a temperature of from 0 to 150°C, according to the following reaction scheme:

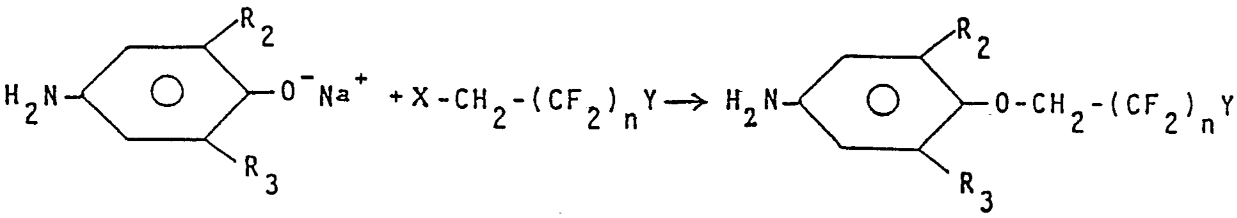

(IV) (V) (III) + NaX

b) reduction, according to known techniques, of a nitroderivative of formula:

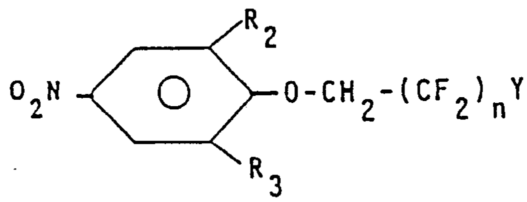

(VI)

The nitroderivatives of formula (VI) may in turn be prepared, according to known methods, by reacting a suitable 4-halonitrobenzene (VII) with a sodium or potassium salt of an alcohol (VIII) in a solvent system consisting of said alcohol (VIII) or in an aprotic apolar solvent, or carrying out the reaction according to the phase transfer technique in the presence of a suitable catalyst at temperatures ranging between 10 and 150°C, according to the following reaction scheme:

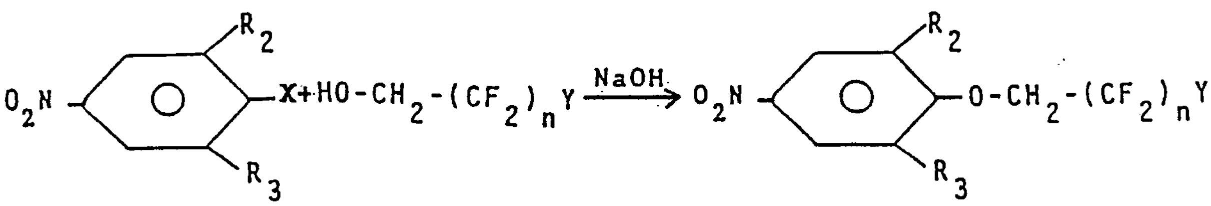


(VII) (VIII) (VI) + NaX

wherein X is halogen.

The alcohols of formula (VIII) are known and may be prepared according to the following reaction scheme:

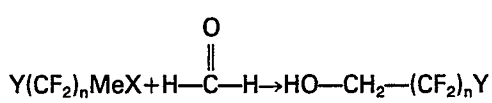

(IX) (X) (VIII)

wherein X is halogen and Me is a metal selected from Zn, Mg and Cu.

Organometallic compound (IX) may be prepared by reacting compound $X(CF_2)_nX$ with Zn, Mg or Cu.

The nitroderivatives of formula (VI), in which at least one of radicals $R_2$, $R_3$ is chlorine, may also be obtained by direct chlorination with $Cl_2$ of the corresponding non-chlorinated nitroderivatives at temperatures ranging between 25 and 150°C and in the presence of a catalyst such as e.g. $FeCl_3$ and $SbCl_5$.

For those skilled in the art, it is a matter of course that different alternative processes may be used for the synthesis of the intermediates and of the end products having formula (I).

An alternative process for the synthesis of the compounds of formula (I) comprises, for instance, reacting a benzamide of formula:

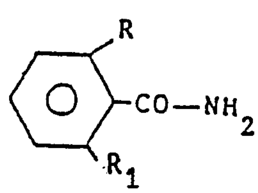

(XI)

with an isocyanate of formula:

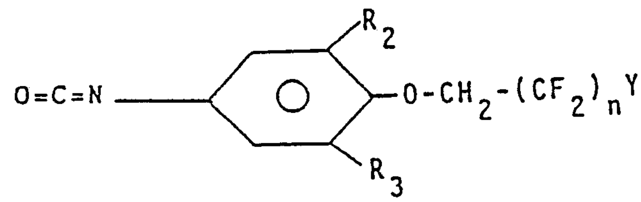 (XII)

This reaction is carried out under conditions similar to those described hereinbefore for the reaction between the benzoyl-isocyanate of formula (II) and the amine of formula (III).

The preparation of the isocyanates of formula (XII) involves, however, the preparation of the amines of formula (III) and their reaction with phosgene. This aspect, together with the consideration that the benzoyl-isocyanates of formula (II) are commercially available, as the amides of formula (XI) are, generally renders the method mentioned hereinbefore, namely the reaction between the compounds of formulae (II) and (III) more preferable.

As mentioned above, the compounds of formula (I) exhibit high insecticide activity, particularly against larvae and eggs of insects. Among these, insects belonging to the orders Lepidoptera, Diptera and Çoleoptera, are especially amenable of being combatted by means of the compounds of formula (I). These orders comprise many species which are important owing to their noxiousness in the agrarian, forestal, civil and veterinary fields.

Therefore, the compounds of formula (I) are suitable for many different utilities, for instance, for protecting agricultural cultivations against infestations of phytophagous insects, for protecting environments infested with mosquitoes and flies, for protecting breeding cattle against cattle parasites, etc. Furthermore, the compounds of formula (I) show a collateral acaricide activity.

For practical use, the compounds of formula (I) may be utilized either as such or, more conveniently, in the form of compositions containing, besides one or more of the compounds of formula (I) as active component, inert solid or liquid carriers and, optionally, other additives.

As with conventional formulations, the compositions according to the present invention may be in the form of wettable powders, emulsifiable concentrates, etc. The amount of active component in these compositions may vary within wide ranges (1—95% by weight), depending on the type of composition and on the intended use.

If required by particular situations or in order to enlarge the spectrum of activity, other active substances such as e.g. known insecticides or acaricides may be added to the compositions.

The amount of active substance (compound of formula I) to be distributed for the insecticide treatments depends on different factors such as, for instance, the type and degree of infestation, the environment infested (agrarian cultivation, basins, water-courses, organic substrates of different nature), the type of the composition employed, climatic and environmental factors, the applicator means used, etc. Generally, amounts of the active substance within the range of from 0.01 to 1 kg/ha are sufficient for a good disinfestation.

The following non-limiting examples are given in order to illustrate the invention.

In these examples the following abbreviations are used in the NMR spectra ($^1$H-NMR):

s = singlet
m = multiplet or unresolved complex signal
b = (broad) = broad signal
ABq = quartet of AB type.

## Example 1

Preparation of N-(2-chloro-benzoyl-N'-[3,5-dichloro-4-(2,2,3,3,4,4,5,5,-octafluoropentyloxy)phenyl]urea (compound No. 1)

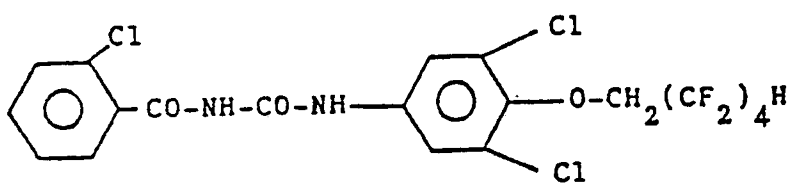

1.0 g of 3,5-dichloro-4-(2,2,3,3,4,4,5,5-octafluoropentyloxy)aniline (prepared according to subsequent example 3), dissolved in 15 ml of anhydrous ether, was introduced into a 50 ml two-neck flask, equipped with a cooler, a dropping funnel and a magnetic stirrer.

Then, 0.7 g of 2-chlorobenzoylisocyanate, dissolved in 5 ml of anhydrous ether, were dripped at room

temperature. The mixture was kept under stirring for 30 minutes, filtered under nitrogen and the precipitate was washed with ether and finally dried under nitrogen.

0.78 g of benzoylurea were obtained, having a melting point of 173—175°C.

Example 2

Starting from the anilines described in subsequent example 3 and using 2-chlorobenzoyl-isocyanate, the following compounds were prepared under conditions similar to those described in example 1:

— compound No. 2: N-(2-chlorobenzoyl)-N'-[4-(2,2,3,3,4,4,5,5-octafluoropentyloxy)phenyl]urea

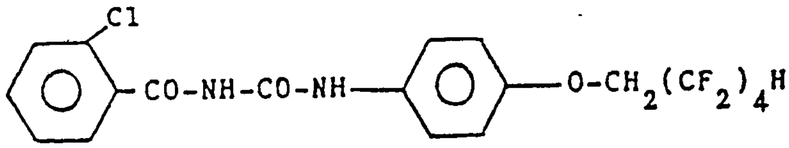

m.p. = 140—142°C.

— compound No. 3: N-(2-chlorobenzoyl)-N'-[3-chloro-4-(2,2,3,3,4,4,5,5-octafluoropentyloxy)-phenyl]urea

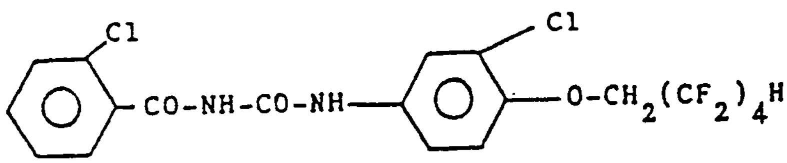

m.p. = 155—157°C.

Additionally, the following compounds were prepared using 2,6-difluorobenzylisocyanate:

— compound No. 4: N-(2,6-difluorobenzoyl)-N'[4-(2,2,3,3,4,4,5,5-octafluoropentyloxy)phenyl]urea

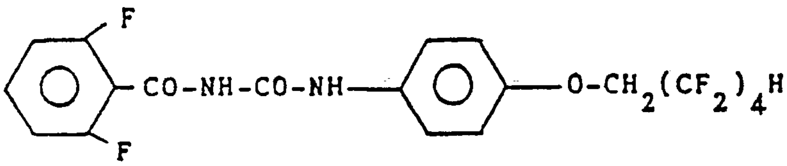

m.p. = 168—170°C.

— compound No. 5: N-(2,6-difluorobenzoyl)-N'-[3,5-dichloro-4-(2,2,3,3,4,4,5,5-octafluoropentyloxy)-phenyl]urea

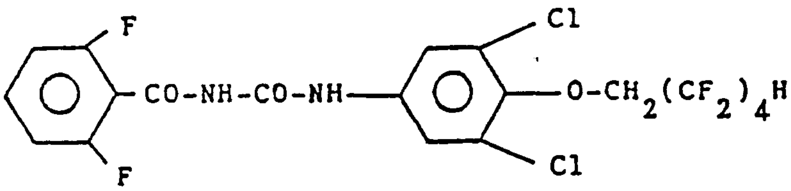

m.p. = 155—157°C.

Example 3

Preparation of intermediate anilines

29.6 millimoles of $SnCl_2 \cdot H_2O$ were introduced into a 50 ml two-neck flask, equipped with a reflux cooler, a dropping funnel and a stirrer, and dissolved in 10 ml of 37% HCl.

Then, 10 ml of a methanolic solution were dripped, containing 5.92 millimoles of 2,6-dichloro-4-nitrophenyl-2,2,3,3,4,4,5,5-octafluoropentylether. The mixture was heated at 60°C for 30 minutes, thereafter cooled down, poured into a 10% solution of NaOH, extracted by means of ethylether and the ethereal extract was dried with $Na_2SO_4$ and finally concentrated. 2 g of 3,5-dichloro-4-(2,2,3,3,4,4,5,5-octafluoro-pentyloxy)aniline were obtained in a yield of 86%.

$^1$H-NMR: 6.5 (s, 2H, arom.); 6.9—6.0—5.15 (tt, 1H, —$CHF_2$); 4.3 (t, 2H, —$CH_2O$); 3.5 (s, 2H, —$NH_2$).

By operating under conditions similar to the ones described hereinbefore and starting from 4-nitrophenyl-2,2,3,3,4,4,5,5-octafluoropentylether, 4-(2,2,3,3,4,4,5,5-octafluoropentyloxy) aniline was obtained in a yield of 95%.

$^1$H-NMR: 6.7 (d, 4H, arom.); 6.8—6.1—5.2 (tt, 1H, —$CHF_2$); 4.35 (t, 2H, —$CH_2O$); 3.5 (s, 2H, —$NH_2$).

Furthermore, starting from 2-chloro-4-nitrophenyl-2,2,3,3,4,4,5,5-octafluoropentylether, 3-chloro-4-(2,2,3,3,4,4,5,5-octafluoropentyloxy)aniline was obtained in a yield of 83.6%.

$^1$H-NMR: 6.9—6.5 (m, 3H, arom.); 66.8—6.1—5.2 (tt, 1H, —$CHF_2$); 4.4 (t, 2H, —$OCH_2$—); 3.5 (s, 2H, —$NH_2$).

Example 8

Determination of the insecticide activity.

Test 1

Residual immediate activity against larvae of *Spodoptera littoralis* (Lepidoptera).

Tobacco leaves were treated by mechanical spraying with a water-acetone solution of the product to be tested, having an acetone content of 10% by volume and containing a surfactant. After complete evaporation of the solvents, the leaves were infested with second instar larvae of Lepidoptera. The infested leaves were kept in a suitably conditioned environment throughout the test.

For comparison, tobacco leaves were infested and kept after having been treated with a water-acetone solution containing 10% of acetone and the surfactant only (control treatment).

Ten days after the infestation and after having renewed at least once the treatment substrate, the number of dead larvae as compared to the control treatment was determined.

Test 2

Activity against larvae of *Aedes aegypti* (Diptera). Plain water (297 ml) was mixed with an acetone solution (3 ml) of the product to be tested in a suitable concentration. 25 Diptera larvae, 4 days of age and suitably fed, were introduced into the obtained solution. For comparison, other larvae were introduced into a water-acetone solution (3 ml acetone, 297 ml plain water) without any active substance.

Every 2-3 days, the number of dead larvae and pupae and of the adults emerging normally from the cocoon, was noted until there occured no more emergence from from the cocoon of the insects in the comparative solution.

The activity of the product under examination was expressed as the percent ratio of dead individuals, compared to the total number of treated individuals.

The insecticide activity in the above mentioned tests was expressed according to the following evaluation:

5 = complete activity (98—100% mortality)
4 = high activity (80—97% mortality)
3 = fair activity (60—79% mortality)
2 = sufficient activity (40—59% mortality)
1 = poor activity (20—39% mortality)
0 = negligible or no activity (0—19% mortality).

The following Table 1 shows the insecticide activity of the tested compounds at the dosages indicated.

TABLE 1

Insecticide activity

| Compound No | Test 1 | | Test 2 |
|---|---|---|---|
| | dosage: 0.001% | dosage: 0.0005% | dosage: 0.01 ppm |
| 1 | 5 | 5 | 5 |
| 2 | 5 | 4 | 5 |
| 3 | 5 | 5 | 5 |
| 4 | 5 | 3 | 5 |
| 5 | 5 | 5 | 5 |
| Reference* | 4 | 3 | 5 |

(*) the reference compound was 1-(2,6-difluorobenzoyl)3--(4-chlorophenyl)urea having the formula:

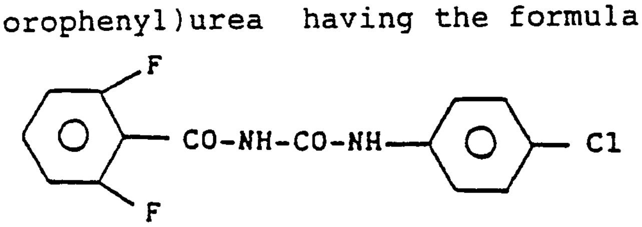

as described in US—A—3 993 908.

**Claims**

1. A compound having the formula

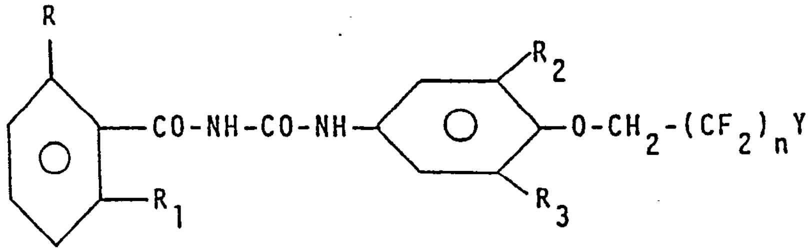

(I)

wherein

R represents chlorine or fluorine;

$R_1$ represents hydrogen, chlorine or fluorine;

$R_2$ and $R_3$, which are the same or different from each other, represent hydrogen or halogen;

Y is hydrogen or fluorine, and

n is an integer of from 1 to 4.

2. A compound according to claim 1, which is N-(2-chlorobenzoyl)-N'-[3,5-dichloro-4-(2,2,3,3,4,4,5,5-octafluoropentyloxy)phenyl]urea.

3. A compound according to claim 1, which is N-(2-chlorobenzoyl)-N'-[4-(2,2,3,3,4,4,5,5-octafluoropentyloxy)phenyl]urea.

4. A compound according to claim 1, which is N-(2-chlorobenzoyl)-N'-[3-chloro-4-(2,2,3,3,4,4,5,5-octafluoropentyloxy)phenyl]urea.

5. A compound according to claim 1, which is N-(2,6-difluorobenzoyl)-N'-[4-(2,2,3,3,4,4,5,5-octafluoropentyloxy)phenyl]urea.

6. A compound according to claim 1, which is N-(2,6-difluorobenzoyl)-N'-[3,5-dichloro-4-(2,2,3,3,4,4,5,5-octafluoropentyloxy)phenyl]urea.

7. A process for the preparation of the compounds according to claim 1, which comprises reacting in an inert solvent and at a temperature of from 0°C up to the boiling point of the reaction mixture, a benzoylisocyanate having the formula:

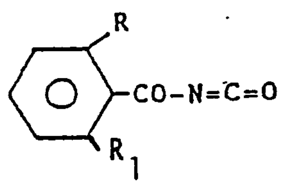

(II)

wherein R and $R_1$ are as defined in claim 1, with an aromatic amine having the formula:

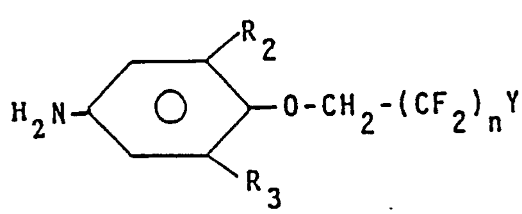

(III)

wherein $R_2$, $R_3$, Y and n are defined as in claim 1.

8. A method for combating infestations of noxious insects, which comprises distributing in the infested zone an effective amount of one or more compounds according to claim 1, either as such or in the form of a suitable composition.

9. Insecticide compositions containing, as active ingredient, one or more compounds according to claim 1 together with solid or liquid, inert carriers and, optionally, other additives.

**Patentansprüche**

1. Verbindung der Formel

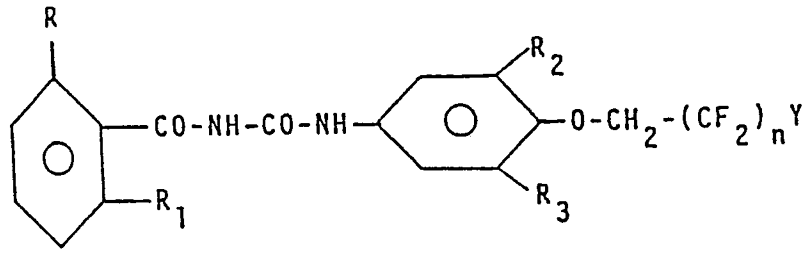

(I)

worin:

R Chlor oder Fluor darstellt;

$R_1$ Wasserstoff, Chlor oder Fluor darstellt;

$R_2$ und $R_3$, die gleich oder voneinander verschieden sind, Wasserstoff oder Halogen darstellen;

Y Wasserstoff oder Fluor ist; und

n eine ganze Zahl von 1 bis 4 ist.

2. Verbindung nach Anspruch 1, die N-(2-Chlorbenzoyl)-N'-[3,5-dichlor-4-(2,2,3,3,4,4,5,5-octafluorpentyloxy)phenyl]harnstoff ist.

3. Verbindung nach Anspruch 1, die N-(2-Chlorbenzoyl)-N'-[4-(2,2,3,3,4,4,5,5-octafluorpentyloxy)-phenyl]harnstoff ist.

4. Verbindung nach Anspruch 1, die N-(2-Chlorbenzoyl)-N'-[3-chlor-4-(2,2,3,3,4,4,5,5-octafluorpentyloxy)phenyl]harnstoff ist.

5. Verbindung nach Anspruch 1, die N-(2,6-Difluorbenzoyl)-N'-[4-(2,2,3,3,4,4,5,5-octafluorpentyloxy)-phenyl]harnstoff ist.

6. Verbindung nach Anspruch 1, die N-(2,6-Difluorbenzoyl)-N'-[3,5-dichlor-4-(2,2,3,3,4,4,5,5-octafluorpentyloxy)phenyl]harnstoff ist.

7. Verfahren zur Herstellung der Verbindungen nach Anspruch 1, umfassend das Umsetzen eines Benzoylisocyanats der Formel

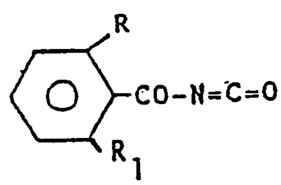

(II)

worin R und $R_1$ wie in Anspruch 1 definiert sind, mit einem aromatischen Amin der Formel

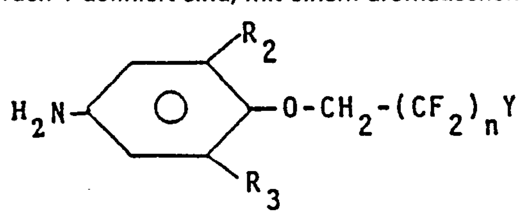

(III)

worin $R_2$, $R_3$, Y und n wie in Anspruch 1 definiert sind, in einem inerten Lösungsmittel und bei einer Temperatur von 0°C bis zum Siedepunkt der Reaktionsmischung.

8. Verfahren zur Bekämpfung des Befalls mit schädlichen Insekten, umfassend das Verteilen einer wirksamen Menge einer oder mehrerer Verbindungen nach Anspruch 1, entweder als solche oder in Form einer geeigneten Zusammensetzung, in den befallenen Gebieten.

9. Insektizid-Zusammensetzungen, enthaltend als aktiven Bestandteil eine oder mehrere Verbindungen nach Anspruch 1 zusammen mit festen oder flüssigen, inerten Trägern und gegebenenfalls anderen Additiven.

**Revendications**

1. Composé ayant la formule suivante:

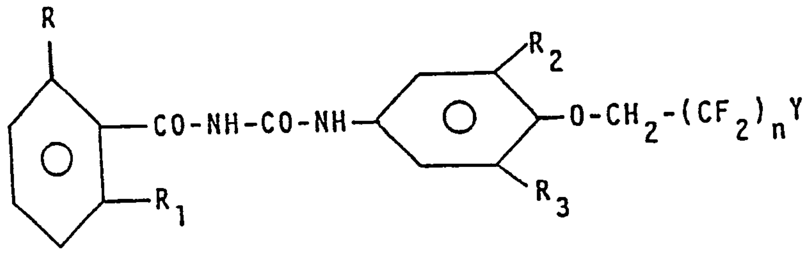

(I)

dans laquelle

R représente un chlore ou un fluor;

$R_1$ représente un hydrogène, un chlore ou un fluor;

$R_2$ et $R_3$, qui sont identiques ou différents, représentent chacun un hydrogène ou un halogène;

Y est un hydrogène ou un fluor; et

n est un entier de 1 à 4.

2. Composé selon la revendication 1, qui est la N-(2-chloro-benzoyl)-N'-[3,5-dichloro-4-(2,2,3,3,4,4,5,5-octafluoropentyloxy)phényl]urée.

3. Composé selon la revendication 1, qui est la N-(2-chlorobenzoyl)-N'-[4-(2,2,3,3,4,4,5,5-octafluoropentyloxy)phényl]urée.

4. Composé selon la revendication 1, qui est la N-(2-chlorobenzoyl)-N'-[3-chloro-4-(2,2,3,3,4,4,5,5-octafluoropentyloxy)phényl]urée.

5. Composé selon la revendication 1, qui est la N-(2,6-difluorobenzoyl)-N'-[4-(2,2,3,3,4,4,5,5-octafluoropentyloxy)phényl]urée.

6. Composé selon la revendication 1, qui est la N-(2,6-difluorobenzoyl)-N'-[3,5-dichloro-4-(2,2,3,3,4,4,5,5-octafluoropentyloxy)phényl]urée.

7. Procédé pour la préparation des composés selon la revendication 1, qui consiste à faire réagir, dans un solvant inerte et à une température comprise entre 0°C et le point d'ébullition du mélange réactionnel, un isocyanate de benzoyle ayant la formule

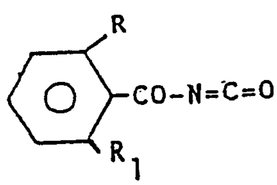

(II)

dans laquelle R et $R_1$ sont tels que définis dans la revendication 1,
avec une amine aromatique ayant la formule suivante:

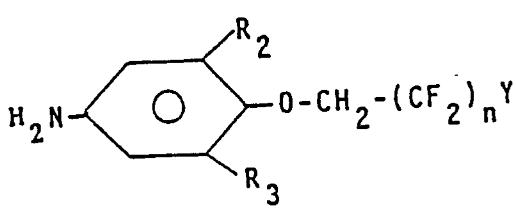

(III)

dans laquelle $R_2$, $R_3$, Y et n sont tels que définis dans la revendication 1.

8. Procédé pour combattre les infestations d'insectes nuisibles, qui consiste à répartir dans la zone infestée une quantité efficace d'un ou plusieurs composés selon la revendication 1, en tant que tels ou sous la forme d'une composition appropriée.

9. Compositions insecticides contenant, en tant que matière active, un ou plusieurs composés selon la revendication 1, en même temps que des supports inertes solides ou liquides, et facultativement d'autres additifs.